# EUROPEAN PATENT APPLICATION

(11) **EP 2 781 232 A1**
(43) Date of publication of application: **24.09.2014**
(21) Application number: 13160395.3
(22) Date of filing: 21.03.2013
(51) Int. Cl.: A61M 15/00, A61M 11/00

(54) **Device for facilitating the inhalation of medicinal products**

(71) Applicant: CA-MI S.R.L., 43010 Pilastro (IT)
(72) Inventor: Apolet, Josek Berek, 20146 Milano (IT); Attolini, Mario, 43037 Lesignano de' Bagni (IT); D'Ettore, Francesco, 43123 Parma (IT); Cogo, Annalisa, 44100 Ferrara (IT)
(74) Representative: Palladino, Saverio Massimo

(57) **Abstract**

The present invention relates to a spacer device for promoting the inhalation of medicinal products that must be administered by inhalation route.

The spacer device (10; 20) is formed by a wall (11; 21) with an essentially ellipsoidal geometry. Said wall has two openings on two opposite faces thereof, a first opening (12) with a shape suitable for connection with an inhalable product source and a second opening (13) shaped in the form of a nozzle (14). The wall is also shaped to form a blind recess (15) or through recess (22), which forms an obstacle (16) that is interposed between said first and second openings. The wall and the obstacle wall together define a chamber (17) in which the inhalable product is nebulised. The part of the obstacle wall facing the nozzle has one or more openings (18) which place the chamber into communication with the outside.

## Description

### Field of the invention

The present invention relates to a device for promoting the inhalation of medicinal products that must be administered by inhalation route, such as for example medicinal products for the symptomatic treatment of asthma or of allergic episodes.

### State of the art

Some disorders or diseases, of the respiratory system in particular, are commonly preventively or curatively treated by inhalation of suitable medicinal products in aerosol form. Known equipment can be used for such purposes, wherein the medicinal product (generally liquid) is introduced into a dedicated slot, and which transforms it into aerosol by means of ultrasound applications for example; these pieces of equipment, however, are only suitable for use in a specific location, for example at home or in hospital.

Some respiratory system diseases, such as asthma or seasonal allergic episodes due to pollens, extemporaneously manifest, even in acute form, outdoors or, in any case, in places where it is not possible to use classic aerosol equipment; in these cases an immediate symptomatic treatment is necessary. The suitable medicinal products are thus packed in dosers in the form of nebulisers.

It has however been verified that these treatments are only truly effective if there is synchrony between the nebulisation action (also simply called "puff") and inspiration by the patient; when these two actions do not occur contemporaneously or almost contemporaneously, a large part of the medicinal product is retained on the walls of the oropharyngeal cavity and on the tongue, thus being lost for the purposes of the treatment; the problem especially manifests in children of up to a few years of age and in the elderly.

To overcome the problem, spacer elements are used, to be applied between the medicinal product nebuliser and the patient's mouth; the principle of operation of these elements is to dilute every single nebulisation in the space (and over time), thus creating a mixture with air having an almost constant composition that is breathed in by the patient, thus untying, at least in part, the amount breathed in from the precise moment of the "puff".

Many types of spacers are known, having different geometries, sizes and functional characteristics.

A widespread spacer is the AeroChamber^{®} (registered trademark of the Canadian company Trudell Medical International) product, available in various versions and sizes suitable for different age groups. This device consists of a cylindrical chamber made of transparent plastic which is interposed between the medicinal product doser and the patient's mouth (or mouth-nose) and it is rather bulky.

Products similar to the AeroChamber^{®} are the Nebuhaler^{®} (AstraZeneca) and the Volumatic (GlaxoWellcome), of approximately ellipsoidal form.

Other geometries of devices that facilitate aerosol inhalation are described in various patent documents.

Patent US 4,534,343 describes a vertical chamber into which the medicinal product is dosed from the top, with a lower opening that acts as a non-return valve to admit air, and a side derivation onto which the tube bearing the mouthpiece or the mask for inhalation by the patient is connected.

Patent application WO 2004/101040 A1 describes a spacer that has, along the axis from the doser to the patient's mouth, an area with a widened cross-section (diffuser), an area with a constant cross-section in which the medicinal product released by the doser is mixed with air, and a part with a narrow cross-section which acts as a mouthpiece.

Patent application GB 2454742 A describes a spacer, which comprises, at approximately half of its length, a movable wall hinged onto an axis transverse to that of the spacer and, immediately downstream of the movable wall (in the direction of the flow of air during inhalation), one or more openings of adjustable size on the wall of the spacer; the purpose of the movable wall is to deflect part of the medicine towards the openings to reduce the inhaled amount in a controlled and adjustable manner (by acting on the size of the openings).

Patent application AU 2011100109 A4 describes a spacer having axial symmetry, which, along the axis that goes from the doser to the patient's mouth, has first areas of cylindrical symmetry having a gradually increasing diameter that are connected to each other by truncated cone walls and thereafter areas of cylindrical symmetry having a gradually decreasing diameter that are in turn connected to each other by truncated cone walls.

All these devices have in common the problem that the formation of a sufficiently diluted and homogeneous mixture between the medicinal product and air is entirely entrusted to the time that the particles of the medicinal product remain in the spacer chamber, so that their efficiency is proportional to the volume; for example, Nebuhaler^{®} and Volumatic devices have chambers with internal volumes of about 750 ml. Consequently, these known devices are not suitable for being carried by the patient at all times, with a view to an extemporaneous use at unforeseeable moments, and are moreover not easily handled by children.

The aim of the present invention is to provide a device for promoting the intake of inhalable products that are to be administered by inhalation route, which affords an inhalation efficacy at least equal to or greater than that of the known devices, but with a reduced volume compared thereto.

### Summary of the invention

These and other aims are obtained with the present invention wit a spacer device between an inhalable product source and a patient's mouth and/or nose having the form of a chamber defined by a generically ellipsoidal wall, wherein: said wall has two openings on two opposite faces thereof, the first opening with a shape suitable for connection with the inhalable product source and the second opening shaped in the form of a nozzle and/or such as to be connectible to a mask holder; said wall is shaped so as to form an obstacle, interposed between said first and second openings, consisting of a blind or through recess on the outer surface of the chamber; and the part of the wall of said obstacle facing said second opening has a series of openings of overall size between 1 and 10 mm², which place the chamber in communication with the outside.

### Brief description of the drawings

- figure 1 shows a device of the invention, according to a first embodiment, in a cross-sectional view along the plane on which the axis that goes from the first opening to the second opening and the axis of the obstacle lie;
- Figure 2 shows, in a view analogous to that of Figure 1, a device of the invention according to a preferred embodiment;
- Figure 3 shows the device of Figure 2 in a cross-sectional view along a plane perpendicular to that of Figure 1, and that contains the axis of the obstacle;
- Figure 4 shows some possible cross-sections of the blind recess or through recess formed in the outer wall of the device of the invention.

### Detailed description of the invention

The device of the invention has the inner chamber of a generically elliptical form, thus characterized by a major axis and two minor axes (the latter are not necessarily of equal length to each other); it is however possible that the three axes are of equal length, thus forming a device in which the inner chamber has a spherical form.

The form of the inner chamber does not necessarily correspond to that of the outer surface of the device; for example, the outer surface of the device could be ergonomically shaped, or could have an essentially parallelepiped form (preferably with rounded edges and vertices) to allow an easier and safer grip by the user.

The elements of the device do not necessarily need to be arranged along the above-defined three axes of the chamber; for example, in the case of ellipsoidal chamber, the axis of the obstacle or the one which joins said first and second openings could not coincide with any of the chamber's three axes; the only necessary condition is that the obstacle be interposed between said first and second opening, thus preventing a direct path between the two.

Finally, even if the most commercially common inhalable product sources are nebulisers of medicinal substances, consisting of pressurised canisters, it is also possible to envisage, as sources of inhalable products, the use of wafers containing (or impregnated with) said products when these easily vaporise in a spontaneous manner. In this case, the first opening can be shaped in the form of housing for a similar wafer, or said housing can consist of a separate element and the first opening can be shaped so as to be easily connectible with said housing; the housing of said wafer (both in the case where it is an integral part of the device, and in the case where it is a separate element to be connected thereto) can be, for example, in the form a small cylindrical chamber, in which the two ends allow an easy passage of air and are equipped with wafer retention elements (nets or filters for example).

Preferably, the device has an inner chamber of ellipsoidal form since this geometry, compared to the spherical geometry, ensures a greater volume for the mixing of the inhalable product with air; additionally, for reasons of constructive simplicity, the axis of the obstacle of the device coincides with one of the axes of the chamber, and the axis that connects said first and second openings essentially coincides with a second axis of the chamber, that is perpendicular to the axis of the obstacle. Lastly, according to an even more preferred geometry, the axes of the obstacle and the one along which said first and second openings are located correspond to the two minor axes of the chamber.

In the following description, for the sake of brevity reference will be made to the more preferred, above-described geometry and to the use of the device in connection to a nebuliser of the medicinal product, it being however understood that the invention is not limited to this particular geometry, and can be realized in practice according to more extensive and generic methods, as previously described.

In the figures, the different parts of the device of the invention are not necessarily to scale; identical numbers in different figures indicate like or corresponding elements.

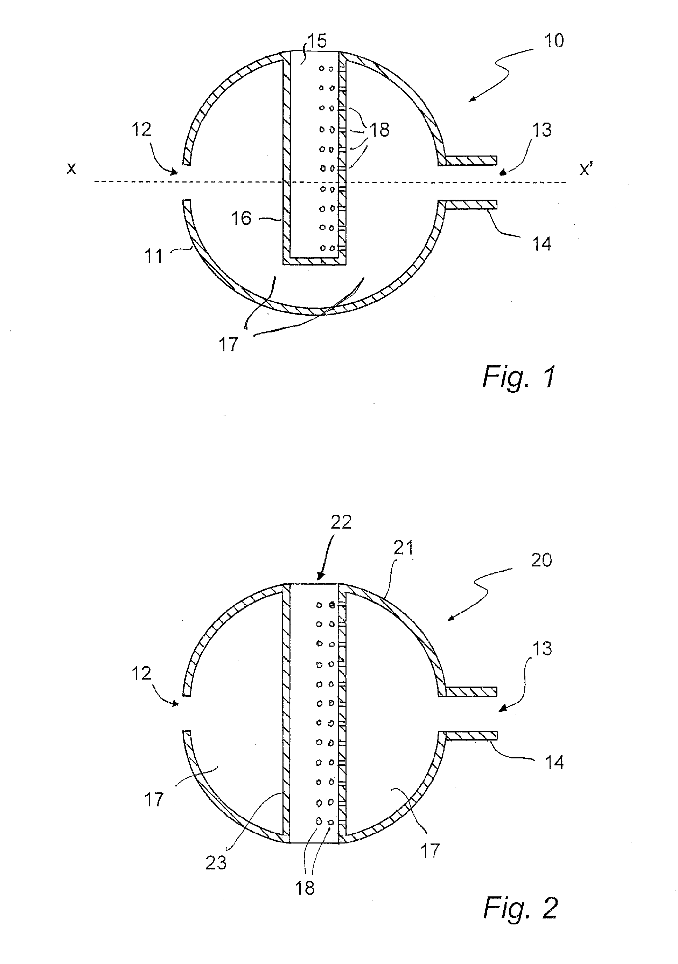

Figure 1 shows a first possible device of the invention, in a cross-sectional view along the plane that is perpendicular to the major axis, and which contains the two minor axes, of the chamber.

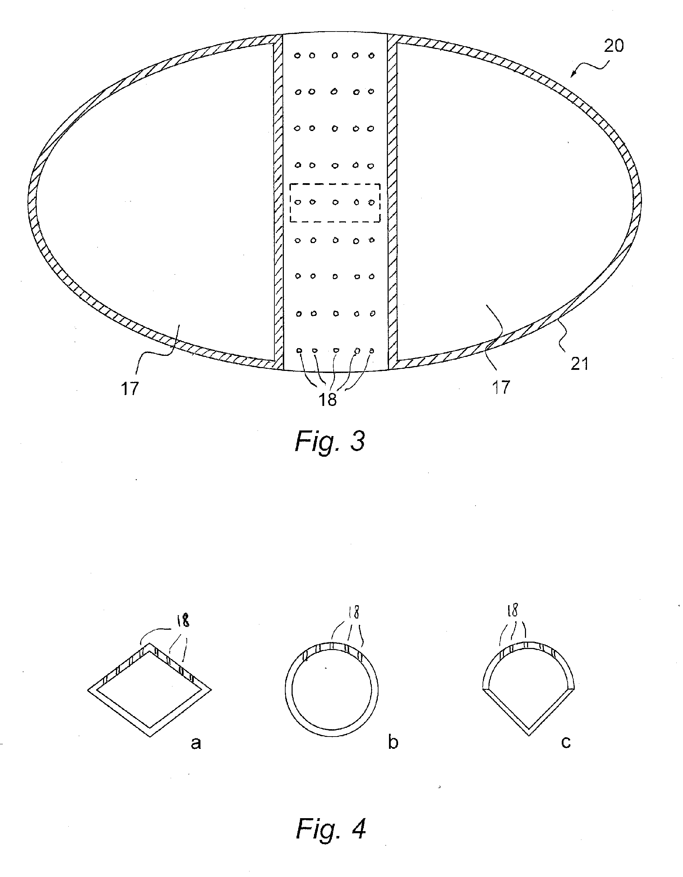

The device, 10, is formed by the wall 11, with an essentially ellipsoidal geometry, which presents, at a first intersection with the minor axis x-x' a first opening 12, shown in the drawing in its simplest possible form; it is however possible that the wall 11 is deformed at the opening 12 so as to result suitable for connection with nebulisers of inhalable products on the market. The wall 11 also has a second opening 13 at the second intersection with the minor axis x-x'; generally the wall 11 is shaped at this opening so as to form a nozzle 14 suitable for the intended use. The wall 11 is also shaped to form a blind recess, 15, which forms an obstacle 16 that is interposed between the openings 12 and 13. The shape of this obstacle is not constraining, in particular as regards its cross-sectional top view (as detailed below with reference to Figure 4), but also in its cross-section in the view of Figure 1, which could be essentially cylindrical or frusto-conical, provided that the necessary condition, that said obstacle prevents a direct path of the aerosol particles from the opening 12 to nozzle 14, is met. The wall 11 and the obstacle wall 16 (joined to each other with continuity) together define the chamber 17 in which the inhalable product is nebulised. The part of obstacle wall 16 facing the nozzle 14 has one or more openings, collectively indicated in the figures as element 18, which place the chamber into communication with the outside. The openings 18 have the aim of allowing air to be drawn from outside the chamber only as a consequence of inhalation, to prevent the chamber itself from creating a vacuum thus making the device difficult to use and allowing the formation of an air-inhalable product mixture that promotes inhalation of the latter. The openings 18 can have different forms; they can, for example, have the form of slots, of a length even equal to the total length of the obstacle 16; or they can have the form of holes of square, rectangular or round cross-section for example. The total surface area of the openings 18 has a maximum value of 30% of the surface of the obstacle wall; this condition ensures that the mixture inspired by the patient during inhalation does not comprise an excessive amount of air originating from outside the device, and thus an effective absorption of the product to be inhaled, without the patient having to exert an excessive inspiration effort.

In addition to meeting the above set out conditions relating to the total surface area thereof, the openings 18 also have individually preferred size. Each individual opening has size not exceeding 4 mm², to prevent the nebulised product from dispensing outside through them; to this end, the cross-section of the openings can be non-constant across the thickness of the obstacle wall 16, and have a greater cross-section in the part facing the recess 15 than in the part facing the inside of the chamber 17. In addition, each opening preferably has size no less than 0.1 mm², both to prevent imposing excessive inspiration efforts on the patient, and so as not to have an excessive number of openings for obtaining the minimum total open surface area as previously indicated. In each device of the invention, the number of openings in the obstacle wall 16 is determined by the specific overall open surface area and by the specific size selected for the individual openings.

The figures show the case of openings 18 in the form of circular holes, but the device of the invention can be produced with any other type of openings, for example in the form of longitudinal slits.

Figures 2 and 3 show a preferred embodiment of the device of the invention, respectively in a view analogous to that of Figure 1 and in a cross-sectional view perpendicular to the axis that joins the opening for connection of the nebuliser to the nozzle.

In this case, the wall 21 of device 20, has a through recess 22 between the two opposite sides of the device itself, which forms a continuous obstacle 23 within the chamber 17. In this case, as shown in Figure 2, the obstacle 23 typically has walls parallel to the axis of the recess (even if it would be possible in this case too to envisage an obstacle having a longitudinal frusto-conical cross-section as in the case of Figure 1).

Figure 3 shows the preferred device of Figure 2, in a view along the x-x' axis of the device in cross-section, and in particular from the point of view of the opening 12; the dotted line of rectangular development in the figure represents the nozzle 14.

Lastly, Figure 4 shows some possible cross-sections of the obstacle 16 or 23; these sections are not necessarily symmetrical with respect to the axis of the recess, and can for example be formed by the joining of two walls that are opposing with respect to the plane perpendicular to axis x-x', having different geometries. The obstacle of Fig. 4a is symmetrical and has a generally rhombohedral cross-section (that can become square when the two diagonals of the rhombus are equal); the obstacle of Fig. 4b is symmetrical, having a circular cross-section (it would also be possible to have a obstacle having an elliptical cross-section); conversely, the obstacle of Fig. 4c has a non-symmetrical cross-section, formed by a part, facing the opening 12 of the device, consisting of two planar walls that form an angle to each other, and by a part, facing the opening 13 of the device and that bears the holes 18, of cylindrical geometry.

The walls that define the chamber 17 (internal surfaces of the wall 11 and of the obstacle 16 or 23) can be made with any material, and preferably, for productive convenience, of a biocompatible and antistatic polymer, with a thickness of between about 1 and 3 mm. On the outer surface of the device there may be present an insert of a different material, for example high "grip" material, to allow a more secure grip by the user.

The size of the devices of the invention is convenient; typically, the major axis of the device is about 8 cm long, while the two minor axes (not necessarily equal to each other, even if this condition is preferable for ease of production) can have a length of between about 4 and 6 cm.

During use, the nebuliser (not shown in the figures) is brought next to or connected to the opening 12, and the medicinal product is nebulised by acting on the appropriate button. The medicine is deflected sideways by the obstacle 16 or 23, which in the side facing the opening 12 has a continuous wall, and is diverted into the lateral parts of the chamber 17 with a turbulent motion that favors mixing with the air present in the chamber. At the moment when the patient performs the inspiration, the nebulised medicine is drawn toward the opening 13 together with the air that enters the chamber through the holes 18, giving rise to a controlled flow of the air-medicine mixture similar to that of the spacers of the prior art, but with markedly smaller size and such that the device of the invention is convenient to carry even in a pocket and also for children to handle.

As has been said, the device of the invention can be used in connection with nebulisers of medicinal products of the traditional type, or with wafers that release curative or aromatic substances. The method of use depends on the type of inhalable product source, and on the overall size of the openings on the obstacle wall 16 (the holes 18, for example) in relation to the size of the chamber and, in the case of sources in the form of nebulisers, on the volume of product nebulised in the chamber 17 at each "puff"; depending on these conditions, it is possible for example, for the patient to inspire after each "puff", or for the patient to take two, three or even more inspiratory acts between one "puff" and another.

## Claims

1. Spacer device (10; 20) between an inhalable product source and a patient's mouth and/or nose, said device internally having the form of a chamber (17) defined by a generally ellipsoidal wall (11; 21), wherein: said wall has two openings on two opposite faces thereof, the first opening (12) with a shape suitable for connection with the inhalable product source and the second opening (13) shaped in the form of a nozzle (14) and/or such as to be connectible to a mask holder; said wall is shaped so as to form an obstacle (16; 23), interposed between said first and second openings, consisting of a blind recess (15) or through recess (22) on the outer surface of the chamber; and the part of the wall of said obstacle facing said second opening has a series of openings (18), of overall size between 1 and 10 mm², which place the chamber in communication with the outside.

2. Device according to claim 1, wherein said inner chamber has a spherical form.

3. Device according to any one of the preceding claims, the external form of which does not correspond to that of the inner chamber and has a contoured ergonomic shape or an essentially parallelepiped form.

4. Device according to any one of the preceding claims, wherein said first opening is shaped in the form of a housing for a wafer that is capable of releasing the inhalable product, or so as to be easily connectible with an outer housing for said wafer.

5. Device according to any one of the preceding claims, wherein said inner chamber (17) has an ellipsoidal form, the axis of the obstacle (16) coincides with a first minor axis of the chamber, and the axis that connects said first and second openings essentially coincides with a second minor axis of the chamber that is perpendicular to that of the obstacle.

6. Device according to any one of the preceding claims, wherein said openings (18) on the obstacle wall (16; 23) have a total surface area not exceeding 30% of the obstacle wall.

7. Device according to any one of the preceding claims, wherein each of said openings (18) on the obstacle wall (16; 23) has size not exceeding 4 mm²

8. Device according to claim 7, wherein each of said openings (18) on the obstacle wall (16; 23) has size not less than 0.1 mm².

9. Device according to any one of the preceding claims, wherein said openings (18) on the obstacle wall (16; 23) are in the form of slots, of a length even equal to the total length of said obstacle, or of holes of square, rectangular or round cross-section.

10. Device according to any one of the preceding claims, wherein the walls that define the chamber 17 are made of a biocompatible and antistatic polymer.
